(19) Europäisches Patentamt
European Patent Office
Office européen des brevets

(11) Veröffentlichungsnummer: **0 517 347 A1**

(12) # EUROPÄISCHE PATENTANMELDUNG

(21) Anmeldenummer: **92250140.8**

(22) Anmeldetag: **04.06.92**

(51) Int. Cl.5: **A61K 31/675**, A61K 31/53, //(A61K31/675,31:195,31:165, 31:485,31:48)

(30) Priorität: **05.06.91 DE 4118740**

(43) Veröffentlichungstag der Anmeldung:
**09.12.92 Patentblatt 92/50**

(84) Benannte Vertragsstaaten:
**AT BE CH DE DK ES FR GB GR IT LI LU NL PT SE**

(71) Anmelder: **SCHERING AKTIENGESELLSCHAFT**
**Berlin und Bergkamen**
**Müllerstrasse 170/178 Postfach 65 03 11**
**W-1000 Berlin 65(DE)**

(72) Erfinder: **Löschmann, Peter Andreas**
**Württembergallee 8**
**W-1000 Berlin 19(DE)**
Erfinder: **Wachtel, Helmut, Dr.**
**Suarezstrasse 22**
**W-1000 Berlin 19(DE)**

(54) **Neue Kombinationspräparate zur Behandlung des Morbus Parkinson, enthaltend NMDA-Antagonisten.**

(57) Die Verwendung von NMDA-Rezeptor-Antagonisten zur symptomatischen Behandlung des Morbus Parkinson und Kombinationspräparate mit synergistischer Wirkung, die NMDA-Rezeptor-Antagonisten enthalten, werden beschrieben.

EP 0 517 347 A1

Die Erfindung betrifft die Verwendung von Antagonisten des N-Methyl-D-Aspartat (NMDA)-Rezeptor-Komplexes oder deren physiologisch verträgliche Salze als Arzneimittel zur symptomatischen Behandlung des Morbus Parkinson sowie pharmazeutische Mittel, die diese Verbindungen enthalten und deren Kombination mit Antiparkinsonmitteln mit synergistischer Wirkung.

Im zentralen Nervensystem von Säugern, einschließlich der Menschen sind hohe Konzentrationen von exzitatorischen Aminosäuren wie Glutamat und Aspartat (Fonnum, F., J. Neurochem. 42: 1-11, 1984) vorhanden. Für die exzitatorischen Aminosäuren existieren verschiedene Rezeptoren, die entsprechend ihrer spezifischen Agonisten als N-methyl-D-Aspartat (NMDA)-, Kainat (KA)- und Quisqualat (QUIS)-Rezeptor bezeichnet werden. Die Quisqualat-Rezeptoren werden nach den spezifischen Agonisten (RS)-$\alpha$-Amino-3-hydroxy-5-methyl-4-isoxazolpropionat auch AMPA-Rezeptoren genannt. Die synaptische Funktion der exzitatorischen Aminosäure L-Glutamat wird auch über NMDA-Rezeptoren vermittelt.

Aus klinischen und tierexperimentellen Befunden gibt es Hinweise, daß es bei der Parkinson schen Krankheit (PD) als Folge des striatalen Mangels an Dopamin zu gesteigerter glutamaterger Neurotransmission in verschiedenen Kernen der Basalganglien kommt. Das Neostriatum (NEO) stellt die Eingangsstruktur der Basalganglien dar: es erhält vom Cortex eine massive glutamaterge Projektion und von der Substantia nigra pars compacta (SNC) die dopaminerge nigrostriatale Bahn, die bei der PD degeneriert. Vom NEO gibt es direkte Bahnen zu den Ausgangskernen der Basalganglien, dem internen Pallidumglied (GPi) und der Substantia nigra pars reticulata (SNR), sowie indirekte, die über das externe Pallidumglied (GPe) und den Nucleus subthalamicus (STH) verlaufen. Der STH erhält eine eigene direkte glutamaterge Innervation vom Cortex; seine zu den Ausgangskernen projizierenden Neurone benutzen ebenfalls L-Glutamat als Transmitter.

Die synaptischen Funktionen von Dopamin im NEO sind komplex. Seine Wirkung auf die zum GPe projizierenden striatalen Neurone ist vorwiegend inhibitorisch, so daß als Folge des striatalen Dopaminmangels, wie er bei der PD vorliegt, die exzitatorischen glutamatergen Einflüsse auf diese Neurone überwiegen. Da sowohl die striatale Bahn zum GPe, als auch die von dort ausgehende zum STH projizierende Bahn inhibitorisch sind, kommt es bei der PD im STH zum Phänomen der Disinhibition mit Anstieg der tonischen Zellaktivität. Über seine glutamatergen Projektionen bewirkt der STH schließlich eine pathologisch gesteigerte neuronale Aktivität in den Ausgangskernen der Basalganglien. Untersuchungen an Tiermodellen der PD zeigen, daß es nach Gabe dopaminerger Substanzen zu einer Normalisierung der gesteigerten exzitatorischen Neurotransmission kommt, die parallel zu der "klinischen" Besserung läuft.

Überraschenderweise zeigte sich, daß NMDA-Rezeptor-Antagonisten, die auch eine neuroprotektive Wirkung auf den $MPP^+$-induzierten Zellverlust in der Substantia nigra haben, allein oder in Kombination mit L-Dopa und direkten Dopaminagonisten eine Wirkung auf das experimentelle Parkinsonsyndrom haben.

Erfindungsgemäß geeignet sind Verbindungen, die eine hohe Affinität zu NMDA-Rezeptoren besitzen und die synaptischen Wirkungen von NMDA aufheben. Solche NMDA-Antagonisten sind beispielsweise:

1.) Kompetitive NMDA-Antagonisten wie 3-(($\pm$)-2-carboxy-piperazin-4-yl)-propyl-1-phosphonsäure (CPP) und Analoga wie beispielsweise CPPene, cis-4-Phosphonomethyl-2-piperidincarbonsäure (CGS 19755) oder auch CGP 40116 und Analoga und 2-Amino-7-phosphonoheptansäure (AP-7) und Analoga.

2.) Antagonisten der Glycin-Bindungsstelle wie beispielsweise Kynurensäure und Analoga, 1-Hydroxy-3-amino-pyrrolidin-2-on (HA-966) und Analoga.

3.) Polyamine wie beispielsweise Spermin und Spermidin und Polyaminantagonisten wie beispielsweise Ifenprodil.

4.) Hemmer der excitatorischen Aminosäuren-Synthese oder der Freisetzung wie z.B. Lamotrigen.

Insbesondere eignen sich erfindungsgemäß zur symptomatischen Behandlung der PD kompetitive NMDA-Rezeptor-Antagonisten.

Die physiologisch verträglichen Salze leiten sich von Alkali- oder Erdalkalimetallen oder den üblichen anorganischen oder organischen Säuren wie Salzsäure, Bromwasserstoffsäure, Schwefelsäure, Phosphorsäure, Zitronensäure, Maleinsäure oder Furmarsäure ab.

Die erfindungsgemäße Wirkung wird am Beispiel des CPP dargestellt:

Auslösung von kontralateralen Rotationen an unilateral Substantia nigralädierten Ratten. Männliche Wistar-Ratten wurden 8 Monate vor dem Experiment durch Injektion von 16 $\mu$g 6-Hydroxydopamin (6-OHDA) in die linke Substantia nigra lädiert. Die Messung der Rotationen erfolgte in automatisierten Rotometern. Die Tiere wurden in Plexiglas-Halbschalen (Durchmesser 40 cm) gesetzt und mechanisch mit einem Winkelschrittgeber verbunden. Die Bewegungen mit und entgegen dem Uhrzeigersinn wurden getrennt in 10-Minuten-Intervallen für 2 Stunden erfasst.

Die Ergebnisse dieser Versuche lassen sich wie folgt zusammenfassen:

Die mit Lösungsmitteln behandelten Tiere zeigen kaum spontane Rotationen. Die Behandlung mit L + Dopa (25 mg/kg i.p. ) und Benserazid (100 mg/kg/i.p.) löst leichte Rotationsbewegungen aus. Werden die Tiere zusätzlich mit CPP (0,025, 0,8, 0,39, 1,56 und 6,25 mg/kg/i.p. ) behandelt, werden die durch L-Dopa induzierten Rotationen dosisabhängig verstärkt. Die Zahl der Rotationen in 2 Stunden ist in der höchsten Dosierung von CPP signifikant von der L-Dopa Behandlung verschieden (Abb. 1).

In gleicher Weise wurden die erfindungsgemäßen Verbindungen am Pinselohräffchen untersucht, bei denen durch das Neurotoxin MPTP ein experimentelles Parkinsonsyndrom erzeugt wurde.

Auch hier zeigt sich, daß die Wirkung von L-Dopa (20 mg/kg/i.p.) und Benserazid (20 mg/kg/i.p. ) durch CPP (0,1, 0,39, 1,56 mg/kg/i.p.) dosisabhängig verstärkt wird (Abb. 2).

Im gleichen Modell wurde CPP (0.1, 0.39, 1.56 mg/kg i.p.) mit den direkten Dopaminagonisten Apomorphin (0.05 mg/kg i.p. ) oder Lisurid (0.1 mg/kg i.p. ) geprüft (Abb. 3 und 4). Auch hier führt die kombinierte Behandlung zu einem signifikantem Synergismus (Varianzanalyse und Tukey Test,* $p < 0.0.1$).

Diese Untersuchungen zeigen, daß Antagonisten der NMDA-Rezeptoren in Dosierungen, die allein ohne Wirkungen sind, nach systemischer Applikation die Wirkung von L-DOPA im 6-DHDA-Rotationsmodell und am MPTP-behandelten Pinselohräffchen verstärken.

Die Blockade der zentralen NMDA-Rezeptoren in Strukturen, die beim Morbus Parkinson eine erhöhte neuronale Aktivität aufweisen, durch Verbindungen mit selektiver und nicht-selektiver Wirkung auf NMDA-Rezeptoren eignet sich zur symptomatischen Behandlung des Morbus Parkinson. Besonders günstig ist die Kombination mit herkömmlichen Antiparkinsonmitteln wie L-DOPA, L-DOPA in Kombination mit Benserazid und Dopaminagonisten wie beispielsweise Lisurid, Bromokriptin, Pergolid, Tergurid, Ropinirol, N-0437 (2[N,n-propyl-N-2-(2-thienyl)ethylamino]-5-hydroxytetralin, Cabergolin sowie Anticholinergika.

Durch Kombination der erfindungsgemäßen Arzneimittel mit herkömmlichen Antiparkinsonmitteln wird die zu verabfolgende Dosis des herkömmlichen Arzneimittels verringert und deren Wirkung gesteigert. Die synergistische Wirkung der Kombinationspräparate ermöglicht daher ein früheres Eintreten des gewünschten Effektes und eine Verminderung der Nebenwirkungen. Die danach beobachtete Wirkung ist ausgeprägter und länger anhaltend als nach alleiniger Gabe der Einzelkomponenten.

Die Erfindung umfaßt auch pharmazeutische Mittel, die die genannten Verbindungen enthalten, deren Herstellung und Kombinationspräparate mit synergistischer Wirkung. Die Arzneimittel werden nach an sich bekannten Verfahren hergestellt, indem man den Wirkstoff mit geeigneten Träger-, Hilfs- und/oder Zusatzstoffen in die Form eines pharmazeutischen Präparates bringt, das für die enterale oder parenterale Applikation geeignet ist. Die Applikation kann oral oder sublingual als Feststoff in Form von Kapseln oder Tabletten oder als Flüssigkeit in Form von Lösungen, Suspensionen, Elixieren oder Emulsionen oder rektal in Form von Suppositorien oder in Form von gegebenenfalls auch subcutan anwendbaren Injektionslösungen erfolgen. Als Hilfsstoffe für die gewünschte Arzneimittelformulierung sind die dem Fachmann bekannten inerten organischen und anorganischen Trägermaterialien geeignet wie zum Beispiel Wasser, Gelatine, Gummi arabicum, Milchzucker, Stärke, Magnesiumstearat, Talk, pflanzliche Öle, Polyalkylenglykole usw. Gegebenenfalls können darüber hinaus Konservierungs-, Stabilisierungs-, Netzmittel, Emulgatoren oder Salze zur Veränderung des osmotischen Druckes oder Puffer enthalten sein.

Die pharmazeutischen Präparate können in fester Form zum Beispiel als Tabletten, Dragees, Suppositorien, Kapseln oder in flüssiger Form, zum Beispiel als Lösungen, Suspensionen oder Emulsionen vorliegen.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis für die NMDA-Rezeptor-Antagonisten beträgt 0,01 - 5000 mg, wobei die Dosis als einmal zu verabreichende Einzeldosis oder unterteilt in 2 oder mehrere Tagesdosen gegeben werden kann. Für die genannten Substanztypen sind die folgenden Einzeldosisbereiche als geeignet anzusehen:

1)

Für kompetitive NMDA-Antagonisten wie AP-7: 10 - 500 mg, vorzugsweise 150 mg, wie CPP und Analoga: 1 - 250 mg, vorzugsweise 25 mg, wie CGS 19755: 0,1 - 100 mg, vorzugsweise 10 mg.

2)

Für Antagonisten der Glycinbindungsstelle wie Kynurensäure und Analoga: 100 - 5000 mg, vorzugsweise 500 mg, wie HA-966 0,01 - 100 mg, vorzugsweise 5 mg.

3)

Für Polyamin-Antagonisten wie Spermin und Spermidin: 100 - 5000 mg, vorzugsweise 500 mg.

In den erfindungsgemäßen Kombinationspräparaten können die Wirkstoffe in einer Formulierung oder auch in jeweils getrennten Formulierungen vorliegen, wobei die gesamte Dosis einmalig verabreicht oder in mehrere Dosen geteilt wird.

Die tägliche Dosis der Wirkstoffe in den Kombinationspräparate beträgt für das herkömmliche Antiparkinsonmittel ca. die Hälfte der üblichen Dosierung, je nach Art und Schwere der Erkrankung sowie Alter und Gewicht des Patienten. Dies gilt auch für die therapeutischen Dosierungen der NMDA-Antagonisten in der Kombination.

**Patentansprüche**

1. Verwendung von NMDA-Rezeptor-Antagonisten oder deren physiologisch verträgliche Salze zur Herstellung eines Arzneimittels zur symptomatischen Behandlung des Morbus Parkinson.

2. Verwendung von NMDA-Rezeptor-Antagonisten oder deren physiologisch verträgliche Salze nach Anspruch 1 in Kombinationspräparaten mit Antiparkinson-Wirkung.

3. Verwendung nach Anspruch 1 und 2 dadurch gekennzeichnet, daß ein kompetitiver NMDA-Rezeptor-Antagonist eingesetzt wird.

4. Verwendung nach Anspruch 1 und 2, dadurch gekennzeichnet, daß der NMDA-Antagonist ein Antagonist der Glycin-Bindungsstelle ist.

5. Verwendung nach Anspruch 1 und 2, dadurch gekennzeichnet, daß der NMDA-Rezeptorantagonist ein Polyamin oder Polyaminantagonist ist.

6. Verwendung nach Anspruch 1 und 2 dadurch gekennzeichnet, daß der NMDA-Rezeptor-Antagonist Lamotrigen ist.

7. Pharmazeutische Mittel enthaltend einen NMDA-Rezeptor-Antagonisten oder dessen physiologisch verträgliches Salz zur symptomatischen Behandlung des Morbus Parkinson.

8. Pharmazeutisches Mittel zur symptomatischen Behandlung des Morbus Parkinson nach Anspruch 7 enthaltend einen NMDA-Rezeptorantagonisten und L-Dopa oder Dopaminagonisten und/oder Anticholinergika und/oder Benserazid.

9. Synergistisch wirkende Kombination zur symptomatischen Behandlung des Morbus Parkinson enthaltend einen NMDA-Rezeptorantagonisten und L-Dopa oder Dopaminagonisten und/oder Anticholinergika und/oder Benserazid.

Abbildung 1

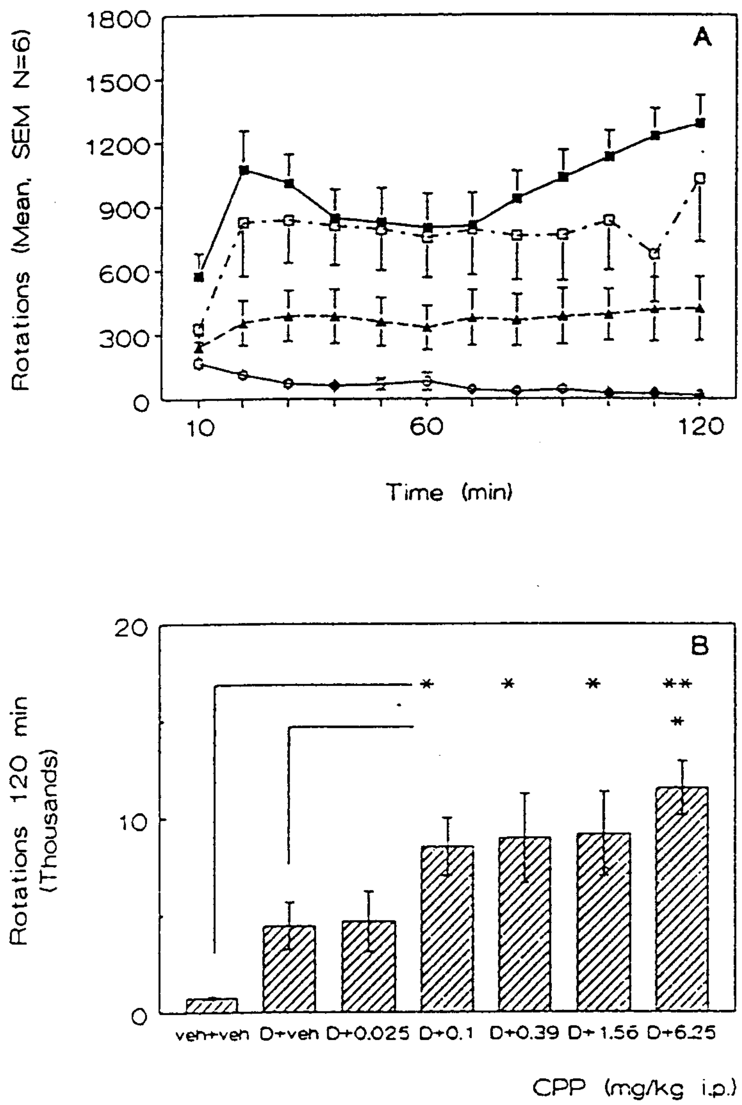
A
Rotations (Mean, SEM N=6)
1800
1500
1200
900
600
300
0
10
60
120
Time (min)
B
Rotations 120 min (Thousands)
20
10
0
veh+veh
D+veh
D+0.025
D+0.1
D+0.39
D+1.56
D+6.25
CPP (mg/kg i.p.)

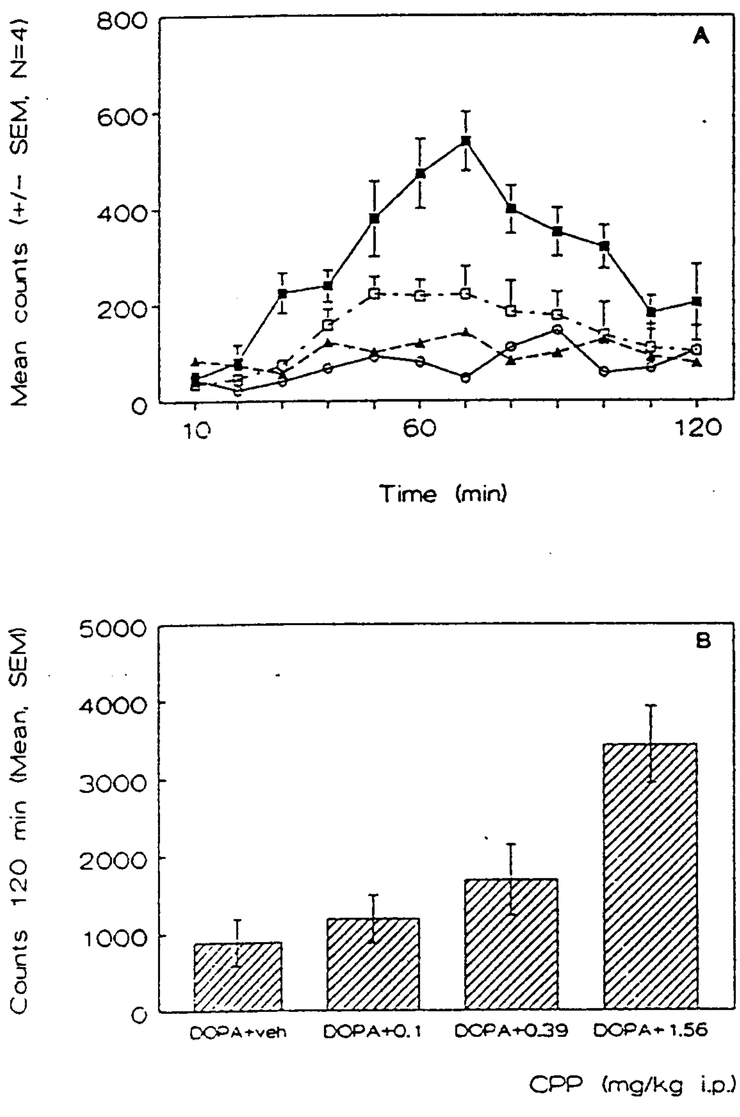
Mean counts (+/- SEM, N=4)
800
600
400
200
0
A
10
60
120
Time (min)
Counts 120 min (Mean, SEM)
5000
4000
3000
2000
1000
0
B
DOPA+veh
DOPA+0.1
DOPA+0.39
DOPA+1.56
CPP (mg/kg i.p.)

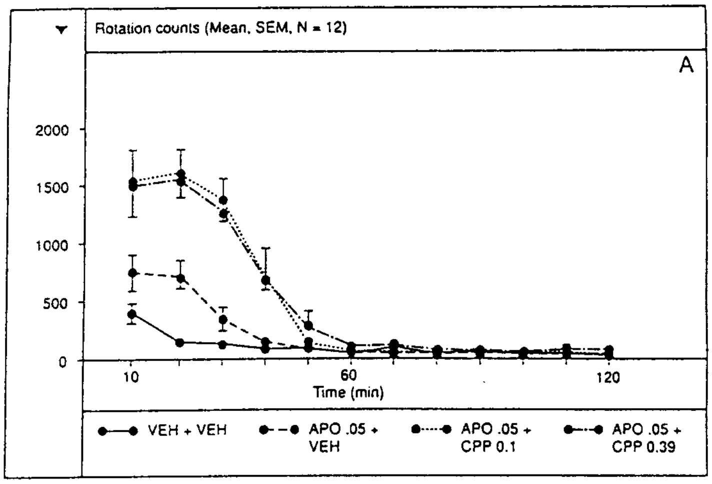
Rotation counts (Mean, SEM, N = 12)
A
2000
1500
1000
500
0
10
60
120
Time (min)
VEH + VEH
APO .05 + VEH
APO .05 + CPP 0.1
APO .05 + CPP 0.39

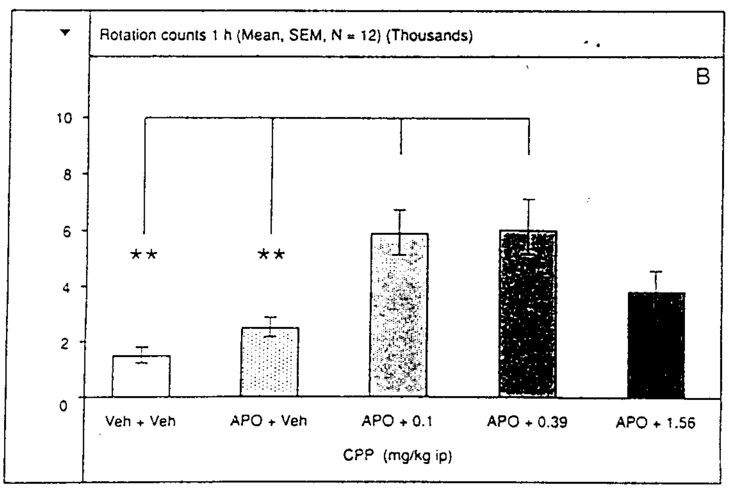
Rotation counts 1 h (Mean, SEM, N = 12) (Thousands)
B
10
8
6
4
2
0
**
**
Veh + Veh
APO + Veh
APO + 0.1
APO + 0.39
APO + 1.56
CPP (mg/kg ip)

Abbildung 4

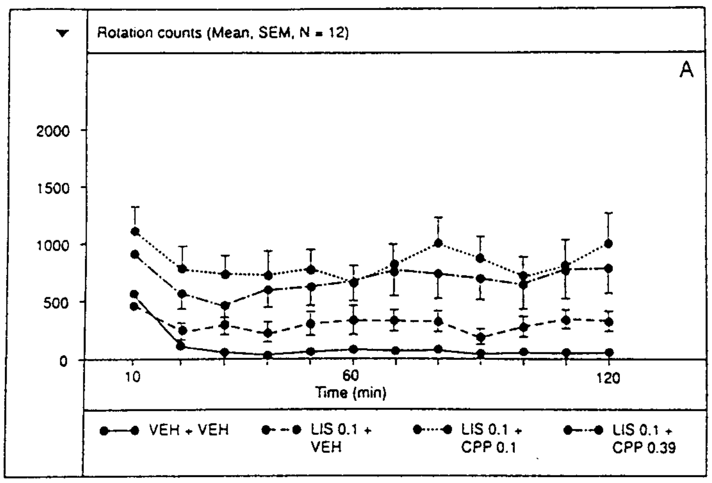
Rotation counts (Mean, SEM, N = 12)
A
2000
1500
1000
500
0
10
60
120
Time (min)
VEH + VEH
LIS 0.1 + VEH
LIS 0.1 + CPP 0.1
LIS 0.1 + CPP 0.39

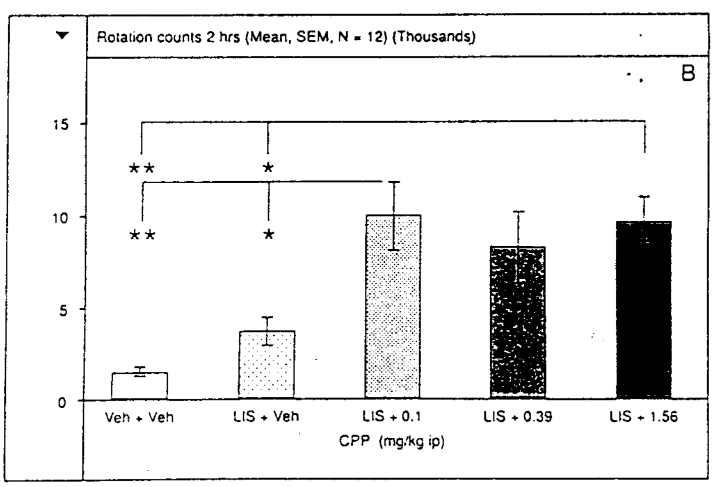
Rotation counts 2 hrs (Mean, SEM, N = 12) (Thousands)
B
15
10
5
0
**
*
**
*
Veh + Veh
LIS + Veh
LIS + 0.1
LIS + 0.39
LIS + 1.56
CPP (mg/kg ip)

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung

EP 92 25 0140

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl.5) |
|---|---|---|---|
| X | ANN. NEUROL., Band 28, Nr. 4, 1990, Seiten 539-546, The American Neurological Association; T. KLOCKGETHER et al.: "NMDA antagonists potentiate antiparkinsonian actions of L-dopa in monoamine-depleted rats" * Insgesamt * --- | 1-9 | A 61 K 31/675<br>A 61 K 31/53 //<br>(A 61 K 31/675<br>A 61 K 31:195<br>A 61 K 31:165<br>A 61 K 31:485<br>A 61 K 31:48 ) |
| X | NATURE, Band 349, Nr. 6308, 31. Januar 1991, Seiten 414-418; L. TURSKI et al.: "Protection of substantia nigra from MPP+ neurotoxicity by N-methyl-D-aspartate antagonists" * Insgesamt * --- | 1-9 | |
| X | TRENDS NEUROSCI., Band 12, Nr. 8, 1989, Seiten 285-286; T. KLOCKGETHER: "Excitatory amino acids and the basal ganglia: implications for the therapy of Parkinson's disease" * Insgesamt * --- -/- | 1-9 | |
| | | | RECHERCHIERTE SACHGEBIETE (Int. Cl.5) |
| | | | A 61 K |

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.
Vollständig recherchierte Patentansprüche:
Unvollständig recherchierte Patentansprüche:
Nicht recherchierte Patentansprüche:
Grund für die Beschränkung der Recherche:

Siehe Blatt -C-

| Recherchenort | Abschlußdatum der Recherche | Prüfer |
|---|---|---|
| DEN HAAG | 14-09-1992 | ORVIZ DIAZ P. |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P0409)

Europäisches Patentamt

EUROPÄISCHER TEILRECHERCHENBERICHT

Nummer der Anmeldung
EP 92 25 0140

| EINSCHLÄGIGE DOKUMENTE | | | KLASSIFIKATION DER ANMELDUNG (Int. Cl.5) |
|---|---|---|---|
| Kategorie | Kennzeichnung des Dokuments mit Angabe, soweit erforderlich der maßgeblichen Teile | Betrifft Anspruch | |
| X | JOURNAL OF NEURAL TRANSMISSION, Band 77, Nr. 1, 1989, Seiten 65-71, Springer-Verlag; M. CARLSSON et al.: "Dramatic synergism between MK-801 and clonidine with respect to locomotor stimulatory effect in monoamine-depleted mice" * Insgesamt * --- | 1-9 | |
| P,X | JOURNAL OF NEURAL TRANSMISSION [P-D Sect.], Band 3, Nr. 3, September 1991, Seiten 203-213, Springer-Verlag; P.-A. LÖSCHMANN et al.: "Synergism of the AMPA-antagonist NBQX and the NMDA-antagonist CPP with L-Dopa in models of Parkinson's disease" * Insgesamt * --- | 1-9 | RECHERCHIERTE SACHGEBIETE (Int. Cl.5) |
| P,X | EP-A-0 434 173 (SCHERING AG)(26-06-1991) * Insgesamt * --- | 1-9 | |
| X | THE CANADIAN JOURNAL OF NEUROLOGICAL SCIENCES, Band 19, Nr. 1 (Supplement), Februar 1992, Seiten 160-162; J. KING CHING TSUI: "Future treatment of Parkinson's disease" * Insgesamt * & 3RD CANADIAN CONFERENCE ON NEURODEGENERATIVE DISEASES, L'Estérel, Québec, 4. - 6. April 1991 --- | 6 | |
| P,X | EPILEPSIA, Band 32, Supplement 1, 1991, Seiten 60-61, Raven Press, New York, US; A.A. SHANDRA et al.: "Effects of lamotrigine on kainate-induced epileptic activity and Parkinsonism" * & 19TH INTERNATIONAL EPILEPSY CONGRESS, Rio De Janeiro, 14. - 19. Oktober 1991 --- -/- | 6 | |

EPO FORM 1503 03.82 (P0412)

Europäisches Patentamt

# EUROPÄISCHER TEILRECHERCHENBERICHT

Nummer der Anmeldung
EP 92 25 0140

| EINSCHLÄGIGE DOKUMENTE | | | KLASSIFIKATION DER ANMELDUNG (Int. Cl.5) |
|---|---|---|---|
| Kategorie | Kennzeichnung des Dokuments mit Angabe, soweit erforderlich der maßgeblichen Teile | Betrifft Anspruch | |
| A | EPILEPSIA, Band 27, Nr. 5, 1986, Seiten 490-497; M.J. LEACH et al.: "Pharmacological studies on lamotrigine, a novel potential antiepileptic drug: II. Neurochemical studies on the mechanism of action" * Insgesamt * ----- | 6 | |
| | | | RECHERCHIERTE SACHGEBIETE (Int. Cl.5) |

EPO FORM 1503 03.82 (P0412)

Unvollständig recherchierte Patentansprüche : 1-9

Die Ausdrücke "NMDA-Rezeptor-Antagonisten", "Antagonist der Glycin-Bindungstelle", "Polyamin oder Polyminantagonist", "Dopaminagonisten" und "Anticholinergika" beinhalten eine grosse Zahl von Verbindungen unterschiedlicher Struktur. Durch derartige Ausdrücke werden chemische Substanzen nicht eindeutig charakterisiert.

Die Recherche musste auf das generelle Konzept, sowie auf die in den Ansprüchen und in den pharmakologischen Beispielen genannten Substanzen beschränkt werden (Richtlinien für die Prüfung im Europäischen Patentamt, Teil B, Kapitel II.7., letzter Satz und Kapitel III.3.7.).

Hierzu muss angemerkt werden, dass Lamotrigine, im Anspruch 6 als "Lamotrigen" bezeichnet, kein Rezeptor-Antagonist im strengen Sinn des Wortes ist, sondern ein Hemmer der Freisetzung excitatorischer Aminosäuren.